(19) **Europäisches Patentamt / European Patent Office / Office européen des brevets**

(11) **EP 3 275 525 B1**

(12) **EUROPEAN PATENT SPECIFICATION**

(45) Date of publication and mention
of the grant of the patent:
**28.08.2019   Bulletin 2019/35**

(21) Application number: **16768965.2**

(22) Date of filing: **25.03.2016**

(51) Int Cl.:
*B01D 39/16* (2006.01)          *A61L 9/16* (2006.01)
*B01D 39/00* (2006.01)          *B03C 3/28* (2006.01)
*B32B 5/26* (2006.01)           *D04H 1/542* (2012.01)
*D04H 1/58* (2012.01)

(86) International application number:
**PCT/JP2016/059751**

(87) International publication number:
**WO 2016/153062 (29.09.2016 Gazette 2016/39)**

(54) **FILTER MATERIAL FOR AIR FILTER**

FILTERMATERIAL FÜR LUFTFILTER

MATÉRIAU DE FILTRE POUR FILTRE À AIR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.03.2015   JP 2015064009**

(43) Date of publication of application:
**31.01.2018   Bulletin 2018/05**

(73) Proprietor: **Toray Industries, Inc.
Tokyo 103-8666 (JP)**

(72) Inventors:
• **YOSHIDA Jun**
  **Otsu-shi**
  **Shiga 520-8558 (JP)**

• **ASADA Yasuhiro**
  **Otsu-shi**
  **Shiga 520-8558 (JP)**
• **MIYOSHI Kengo**
  **Otsu-shi**
  **Shiga 520-8558 (JP)**

(74) Representative: **Hoefer & Partner Patentanwälte
mbB
Pilgersheimer Straße 20
81543 München (DE)**

(56) References cited:
**WO-A1-2009/055047          DE-A1-102012 220 546
JP-A- S51 134 475           JP-A- 2007 130 632
JP-A- 2009 046 776          JP-A- 2013 215 251
JP-A- 2013 215 251          JP-A- 2013 220 375**

**Description**

TECHNICAL FIELD

The present invention relates to an air filter medium.

BACKGROUND ART

With the recent trend toward higher air tightness in housing environments, the surrounding living environments are changing considerably and there is a growing desire for improvements in air environment. A measure for improving an air environment is hence being taken in which an air filter is installed in a room of, for example, a dwelling to collect substances that are harmful and unnecessary to the indoor environment, such as the dust and smoke contained in the indoor air, and to adsorptively remove odors and the like, thereby cleaning the air.

An air filter medium is used in the above-described air filter. A generally and extensively known process for producing an air filter medium is, for example, one in which an adsorbent such as activated carbon is fixed with a polyethylene resin, which is a thermoplastic resin, or the like to form a deodorization layer and this layer is sandwiched from both sides between sheets of fabric, such as nonwoven fabric, woven fabric, or knit fabric, to obtain an air filter medium (see, for example, Patent Document 1). However, such configurations have had a problem in that the fabric itself is flammable in many cases and the air filter medium has insufficient flame retardancy.

Patent Document 2 discloses an adsorption/decomposition sheet in which poly(vinyl chloride)-based fibers are used as fibers having excellent flame retardancy for configuring the fabric in an amount of 60-80% by weight based on the fibrous base, in order to improve the flame retardancy of the fabric itself to be used in air filter media. However, it has become known in recent years that halogen (in particular, chlorine) compounds emit harmful substances such as dioxins upon incineration, etc. Because of this, use of these compounds is coming to be prohibited globally.

Various flame retardants including phosphorus-compound flame retardants and inorganic flame retardants are hence being investigated as flame retardants to be incorporated into the fabric. For example, Patent Document 3 described a flame-retardant filter employing a phosphorus-compound flame retardant.

BACKGROUND ART DOCUMENT

PATENT DOCUMENT

Patent Document 1: JP-A-2004-358389 Patent Document 2: JP-A-10-212685 Patent Document 3: JP-A-2000-126523

SUMMARY OF THE INVENTION

PROBLEMS THAT THE INVENTION IS TO SOLVE

However, the flame-retardant filter disclosed in Patent Document 3 has a drawback in that phosphorus-compound or inorganic flame retardants are low in flame-retarding effect per unit adhesion amount by mass and it is necessary to incorporate the flame retardant in a large amount in order to obtain sufficient flame retardancy, resulting in the necessity of increasing the amount of the binder to be incorporated for adhering the flame retardant to the fabric. In this case, even if excellent flame retardancy is obtained, this flame-retardant filter has reduced air permeability and a high pressure loss. Furthermore, in that case, since the binder also is flammable, the incorporation thereof in a larger amount may result in a possibility that even the flame retardancy might not be sufficiently exhibited. Document WO 2009/055047 A1 discloses an air permeable laminate comprising two fibrous sheets and heat-expandable graphite particles and a binder disposed in at least the inter-laminar space between the fibrous sheets.

Accordingly, an object of the present invention is to provide an air filter medium which has excellent flame retardancy and a low pressure loss.

MEANS FOR SOLVING THE PROBLEMS

In order to solve the above-described problem, an air filter medium according to claim1 is suggested.

ADVANTAGE OF THE INVENTION

The present invention according to claim 1 provides an air filter medium having excellent flame retardancy and a low pressure loss.

BRIEF DESCRIPTION OF THE DRAWINGS

[Fig. 1] Fig. 1 is a diagrammatic slant view of one embodiment of the air filter medium of the present invention, which has burned partly and in which the heat-expandable particles in the area have expanded.
[Fig. 2] Fig. 2 is a diagrammatic cross-sectional view of the air filter medium shown in Fig. 1, taken on the line A-A.

MODE FOR CARRYING OUT THE INVENTION

The present invention will be explained below in detail, but the present invention should not be construed as being limited to the range explained below, unless the present invention departs from the gist thereof.

The air filter medium of the present invention includes: a plurality of stacked fibrous sheets; and heat-expandable particles and a binder disposed in at least one of interlaminar spaces between adjacent layers of the fibrous sheet.

Heat-expandable particles, even when added in a small amount, can render the flame retardancy of the air filter media excellent, although details thereof will be described later. This means that the binder need not be used in a large amount for fixing the heat-expandable particles to the air filter media. Because of this, excellent flame retardancy can be imparted to an air filter medium without lowering the air permeability, which is an important property for air filter media.

The air filter medium of the present invention includes a plurality of stacked fibrous sheets so as to have an excellent efficiency of dust collection. As will be described later, the fibrous sheets include natural fibers or synthetic fibers and are hence flammable. In addition, there are often cases where it is difficult to add a flame retardant to the fibers for constituting the fibrous sheets because the addition thereof results in, for example, a decrease in spinnability and where even when a flame retardant can be added, the content thereof is too low.

In the air filter medium of the present invention, a flammable binder is used for bonding at least some of the fibrous sheets. Although the air filter medium of the present invention has such a configuration, this air filter medium has excellent flame retardancy due to the use of heat-expandable particles therein.

First, the heat-expandable particles to be used in the air filter medium of the present invention are explained. The term "heat-expandable particles" herein means a particulate substance which itself has flame retardancy and which expands upon heating. Examples thereof include lamellar inorganic compounds, flameproofed microcapsules, and hydrous minerals. From the standpoints of excellent expansibility and no inclusion of any flammable resin, it is preferred to use a lamellar inorganic compound as the heat-expandable particles.

The term "lamellar inorganic compound" herein means a lamellar inorganic substance having an intercalation compound therein, and examples of the lamellar inorganic compound include expandable graphites, montmorillonite, and lamellar perovskite compounds. Examples of the lamellar inorganic substance include graphites, metal oxides, phosphoric acid salts with metals, clay minerals, and silicic acid salts. Examples of the intercalation compound include compounds which gasify upon heating, such as sulfuric acid and nitric acid.

Preferred of such heat-expandable particles are expandable graphites, with which it is possible to design a high expansion initiation temperature and a high expansion ratio. In cases when heat-expandable particles having a higher expansion initiation temperature are employed, the heat-expandable particles have excellent handleability, for example, during the production of the air filter medium because these heat-expandable particles do not expand even when heated during the production of the air filter medium and do expand only upon heating due to burning. By heightening the expansion ratio, the flame retardancy can be improved.

The term "expandable graphite" herein means a graphite into which sulfuric acid, nitric acid, or the like has been intercalated. Although the graphite to be used as a raw material is not particularly limited, a graphite in which the crystals have highly grown is preferred, such as natural graphites, kish graphite, and pyrolytic graphites. Natural graphites are preferred of these from the standpoint of a balance between impartation of flame retardancy and profitability.

Examples of the natural graphites include flake natural graphite. In the case of producing an expandable graphite from this natural graphite, an intercalation compound which evolves a gas upon heating is formed between the flake layers of the natural graphite. Upon application of heat to the expandable graphite obtained from the natural graphite, a gas generates from the intercalation compound and the flake natural graphite expands due to the evolved gas to form a carbonized wall which is stable to heat, although details thereof will be described later. It is presumed that this carbonized wall prevents the flame and heat from being transmitted from the burned area to the unburned area to thereby impart excellent flame retardancy to the air filter medium of the present invention.

Even when the heat-expandable particles are added in a small amount, the air filter medium is able to have sufficient flame retardancy. It is hence presumed that even when the heat-expandable particles are added in a small amount, a carbonized wall having a size suitable for preventing the flame or heat from being transmitted from the burned area to the unburned area is formed.

The heat-expandable particles to be used in the air filter medium of the present invention have an expansion initiation temperature of preferably 150°C or higher, more preferably 200°C or higher, especially preferably 300°C or higher. In cases when the expansion initiation temperature thereof is within that range, these heat-expandable particles do not expand upon heating during the production of the air filter medium and do expand only in proper cases, e.g., burning. Although there is no particular upper limit, the expansion initiation temperature thereof is preferably 800°C or lower because the expansion needs to begin upon heating at around the combustion temperature. The expansion initiation temperature of the heat-expandable particles can be regulated, for example, by selecting an intercalation compound.

Next, it is preferable that the heat-expandable particles expand, upon heating (specifically to 800-1,000°C), in an expansion ratio of 50 or higher along the C-axis direction, which is perpendicular to the cleavage plane of the graphite. The heat-expandable particles more preferably have an expansion ratio of 100 or higher, and especially preferably have an expansion ratio of 150 or higher.

By employing heat-expandable particles having an expansion ratio of 50 or higher, the flame retardancy of the air filter medium which includes the heat-expandable particles is further improved. Although there is no particular upper limit, the expansion ratio thereof is preferably 500 or less from the standpoint of availability. The expansion ratio of the heat-expandable particles can be regulated, for example, by changing the content of the intercalation compound. The expansion initiation temperature is a temperature at which the heat-expandable particles, when heated under certain conditions, come to have a volume that is 1.1 time the original volume thereof.

According to the invention the heat-expandable particles in an unexpanded state have a number-average particle diameter of 100 $\mu$m to 800 $\mu$m. The lower limit thereof is more preferably 200 $\mu$m or larger, and the upper limit thereof is more preferably 600 $\mu$m or smaller.

By regulating the number-average particle diameter of the heat-expandable particles in an unexpanded state to 100 $\mu$m or larger, sufficient flame retardancy can be imparted to the air filter medium. In particular, reducing the content of particles each having a particle diameter less than 100 $\mu$m makes it easy to prevent dust scattering and is hence preferred from the standpoint of operation environment. Meanwhile, by regulating the number-average particle diameter of the heat-expandable particles in an unexpanded state to 800 $\mu$m or smaller, diffusing unevenness can be further reduced when diffusing the heat-expandable particles and, hence, the resultant air filter medium can be made to have a reduced pressure loss.

The number-average particle diameter is a value obtained by examining the particles with a scanning electron microscope (SEM) to measure the maximum diameter and minimum diameter of each particle, taking the average of these as the particle diameter, and arithmetically averaging such particle diameters of 100 particles.

Next, the content of the heat-expandable particles is preferably 0.5-30% by mass based on the whole air filter medium, which is taken as 100% by mass. By regulating the content of the heat-expandable particles to 0.5% or higher, the air filter medium can be made to form a carbonized layer having a size more suitable for isolating the unburned portion of the air filter medium from the burned portion of the air filter medium. The flame retardancy of the air filter medium is hence rendered even better. From this standpoint, the lower limit of the content thereof is more preferably 1% by mass or higher, especially preferably 3% by mass or higher.

Meanwhile, by regulating the content of the heat-expandable particles to 30% by mass or less, the amount of the binder necessary for adding the heat-expandable particles to an air filter medium can be further reduced and an air filter medium having excellent flame retardancy and a low pressure loss can be obtained. From this standpoint, the upper limit of the content thereof is preferably 20% by mass or less, more preferably 15% by mass or less, even more preferably 10% by mass or less. By regulating the content thereof so as to be within that range, an air filter medium which has high flame retardancy and a low pressure loss can be obtained.

It is necessary that the air filter medium of the present invention includes a plurality of stacked fibrous sheets and that at least one of the interlaminar spaces between adjacent layers of the fibrous sheets has heat-expandable particles and a binder therein. In the air filter medium having such configuration, the heat-expandable particles are sandwiched between the fibrous sheets and the heat-expandable particles are fixed to the air filter medium with the binder. Consequently, the heat-expandable particles can be inhibited from shedding from the air filter medium.

In the air filter medium of the present invention, the interlaminar space(s) may be the only portion(s) where heat-expandable particles are present, or portions other than the interlaminar spaces, such as, for example, the fibrous sheets, may contain heat-expandable particles therein, unless the effect of the present invention is not lessened thereby.

In the case where the air filter medium includes three or more stacked fibrous sheets and has two or more interlaminar spaces, then at least one of these two or more interlaminar spaces may have heat-expandable particles and a binder therein or all the interlaminar spaces may have heat-expandable particles and a binder therein, although details thereof will be described later.

Details of the mechanism by which the air filter medium of the present invention exhibits excellent flame retardancy are unclear. However, a presumed mechanism whereby the flame retardancy is exhibited is explained below using the drawings.

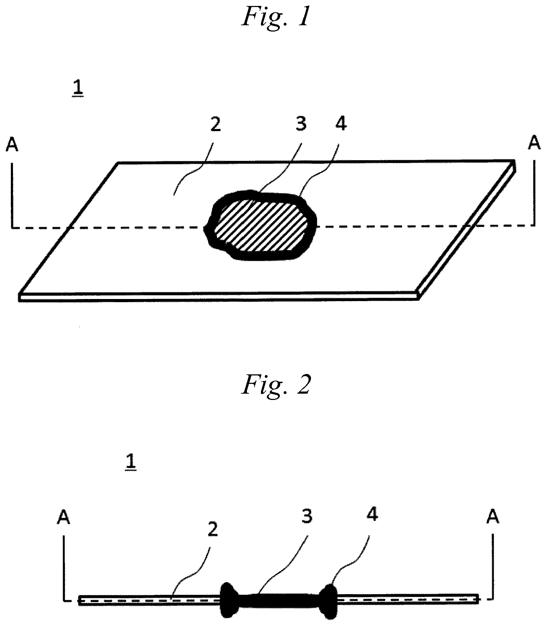

Fig. 1 shows a slant view of an air filter medium which has burned partly and in which the heat-expandable particles in the area have expanded. Fig. 2 shows a cross-sectional view of the air filter medium shown in Fig. 1, taken on the line A-A. In case where some of the air filter medium 1 of the present invention has caught fire, the heat-expandable particles present in the portion of the air filter medium 1 which has caught fire and in the periphery thereof expand due to the heat of the flame, thereby forming a carbonized wall 4 so as to surround the portion which has caught fire. It is presumed that this carbonized wall 4 isolates the unburned portion 2 of the air filter medium 1 from the burned portion 3 of the air filter medium to inhibit the fire from spreading on the air filter medium. The filter medium is presumed to thus exhibit excellent flame retardancy.

Next, the air filter medium of the present invention is explained. It is preferable that the air filter medium of the present invention has a softness of 0.1-20 mN. By regulating the softness thereof to 0.1 mN or higher, the filter medium can be made to have excellent suitability for pleating and be inhibited from suffering a pleat shape deformation due to wind pressure and, hence, an air filter having a low pressure loss can be obtained. Meanwhile, by regulating the softness thereof to 20 mN or less, the filter medium is rendered easy to bend into pleats. The lower limit thereof is more preferably 1 mN or higher, and the upper limit thereof is more preferably 15 mN or less.

A desired value of softness can be imparted by selecting a fiber production method and a fiber diameter or regulating the amount of a binder to be used, etc., or by employing two or more of such techniques in combination. The term "softness" herein means the softness measured by the Gurley method as provided for in JIS L1096, method A (year 2010) (Gurley method).

It is preferable that the air filter medium of the present invention has a thickness of 0.1 mm to 3 mm. The lower limit thereof is more preferably 0.3 mm or larger, especially preferably 0.5 mm or larger. The upper limit thereof is more preferably 2.5 mm or less, especially preferably 2.0 mm or less. By regulating the thickness thereof so as to be within that range, not only the pressure loss of the air filter medium can be further reduced but also the filter medium can be made to have better processability regarding pleating, etc.

The pressure loss of the air filter medium of the present invention, when measured at a surface wind velocity of 10.8 cm/sec, is preferably 150 Pa or less, more preferably 100 Pa or less. The air filter medium of the present invention can be made to have a desired value of pressure loss within that range, in terms of pressure loss as measured at a surface wind velocity of 10.8 cm/sec, by suitably selecting a basis weight of the air filter medium, form of the fibrous sheets, diameter of the fibers to be used, etc. or by employing two or more of these techniques in combination.

Next, the fibrous sheets included in the air filter medium of the present invention are explained. The air filter medium of the present invention includes a plurality of stacked fibrous sheets. The air filter medium of the present invention hence can have a configuration in which three fibrous sheets have been stacked and which has two interlaminar spaces, a configuration in which four fibrous sheets have been stacked and which has three interlaminar spaces, a configuration in which five fibrous sheets have been stacked and which has four interlaminar spaces, etc. The plurality of fibrous sheets may be the same or different. From the standpoint of further enhancing the strength of adhesion between the fibrous sheets, it is preferable that the interlaminar spaces are fixed with a binder.

Examples of the form of each fibrous sheet included in the air filter medium of the present invention include nonwoven fabric, woven fabric, net, and paper. It is preferable that all the fibrous sheets included in the air filter medium of the present invention are nonwoven fabric among these, from the standpoint that nonwoven fabric is excellent in terms of the efficiency of dust collection and suitability for pleating.

The fibers to be used in the fibrous sheets included in the air filter medium of the present invention can be any of a wide range of conventionally known, natural fibers and synthetic fibers. Preferred of these are thermoplastic fibers which can be produced by melt spinning. Examples of thermoplastic resins for constituting synthetic fibers include polyesters, polyamides, polyolefins, acrylics, Vinylon, polystyrene, poly(vinyl chloride), poly(vinylidene chloride), and poly(lactic acid). A suitable resin can be selected according to applications, etc. A plurality of thermoplastic resins may be used in combination.

From the standpoints that thermoplastic fibers can be produced by melt spinning and that thermoplastic resins have excellent spinnability, it is preferable that at least one of the fibrous sheets included in the air filter medium is nonwoven fabric including thermoplastic fibers as a main component. The expression "as a main component" means that thermoplastic fibers are contained in an amount exceeding 50% by mass based on the whole nonwoven fabric, which is taken as 100% by mass. The content of the thermoplastic fibers is more preferably 60% by mass or higher, even more preferably 80% by mass or higher, especially preferably 100% by mass.

From the standpoint of further elevating the dust collection efficiency of the air filter medium, it is preferable that at least one of the fibrous sheets included in the air filter medium is nonwoven electret fabric.

The fibers to be used in the fibrous sheets included in the air filter medium of the present invention each can have any configuration such as the single type, core/sheath type composite fiber, or side-by-side type composite fiber. The cross-sectional shape of the fiber is also not particularly limited, and may be not only a circular shape but also a so-called noncircular cross-sectional shape such as an elliptic shape, triangular shape, star shape, T shape, Y shape, leaf shape, etc. Also usable are fibers having voids in the surface or fibers having a branched structure. According to need, two or more kinds of those fibers can be mixed and used. Crimped fibers may be used in order to enhance the air permeability.

As stated above, the plurality of fibrous sheets included in the air filter medium of the present invention may be the same. It is, however, preferable that the fibrous sheets are fibrous sheets which are different from each other and which have different properties, because use of different fibrous sheets can further improve properties of the air filter medium, such as the dust collection efficiency and pressure loss.

Here, an air filter medium which includes two stacked fibrous sheets that differ from each other and in which the interlaminar space between these fibrous sheets has heat-expandable particles and a binder therein is shown as one embodiment of the air filter medium of the present invention to explain such two different fibrous sheets usable in the air filter medium of the present invention.

In this case, the fibrous sheet located upstream along the direction of air passing is referred to as fibrous sheet A, and the fibrous sheet located downstream from the fibrous sheet A is referred to as fibrous sheet B, for convenience. In this case, the fibrous sheet A and/or the fibrous sheet B itself may be a stack of two or more sheets of nonwoven fabric. With respect to the direction of air passing, in cases when the air filter medium is mounted as a filter unit in, for example, an air cleaner and air is passed therethrough, then the air inflow side is defined as upstream side and the air outflow side is defined as downstream side.

Since heat-expandable particles are disposed in the interlaminar space between the fibrous sheet A and the fibrous sheet B in this air filter medium, the heat-expandable particles can be inhibited from shedding from the air filter medium.

Furthermore, since the fibrous sheet B and the fibrous sheet A differ from each other in performance or property, this air filter medium of the present invention shows even better performance such as, for example, dust collection efficiency. Specifically, a fibrous sheet A which is stiffer, bulkier, and coarser in opening size than a fibrous sheet B is superposed on the fibrous sheet B, and this air filter medium is set in, for example, an air cleaner so that the fibrous sheet A is located on the upstream side along the direction of air passing. As a result, due to the high stiffness of the fibrous sheet A, the air filter medium is more effectively inhibited from deforming even when air is passed at a high rate and the pressure loss can be further reduced.

Meanwhile, in cases when a fibrous sheet B which is finer in opening size than a fibrous sheet A is superposed on the fibrous sheet A and this stack is set as an air filter medium in, for example, an air cleaner so that the fibrous sheet B is located on the downstream side along the direction of air passing, then dust particles which are too small to be collected with the fibrous sheet A can be collected with the fibrous sheet B. Thus, the dust collection efficiency of the air filter medium can be further heightened.

Furthermore, in this case, large dust particles that may clog the fibrous sheet B are collected with the fibrous sheet A, which is bulky and coarse in opening size, without allowing the large dust particles to reach the fibrous sheet B. The fibrous sheet B is thus inhibited from being clogged, and the product life of the air filter medium can hence be further prolonged.

The fibrous sheet A may be nonwoven fabric, woven fabric, net, paper, or the like. It is preferable that the fibrous sheet A is nonwoven fabric among these, from the standpoint that nonwoven fabric is excellent in terms of the efficiency of dust collection and suitability for pleating. The nonwoven fabric can be obtained, for example, by a conventional method for nonwoven-fabric production.

Examples of nonwoven fabric usable as the fibrous sheet A include: nonwoven fabric obtained by subjecting a fiber web obtained by a wet-laid paper method and a dry method to processing by a chemical bonding method, thermal bonding method, needle punching method, water-jet punching method, or the like to bond the fibers to themselves; and nonwoven fabric obtained by a direct spinning method such as a spunbonding method or a melt blowing method. In particular, the wet-laid paper method has advantages over the dry method in that since fibers are dispersed and then formed into a sheet, not only the nonwoven fabric has reduced unevenness in opening size but also fibers of multiple kinds differing in property can be mixed together in any desired proportion to produce nonwoven fabric therefrom, making it possible to obtain a fibrous sheet A having excellent air permeability, which is a basic property required of air filter media.

From the standpoint shown above, it is more preferable that the fibrous sheet A is wet-laid nonwoven fabric obtained by a wet-laid paper method. From the standpoint of using a highly stiff fibrous sheet as the fibrous sheet A, it is especially preferred to employ wet-laid chemical-bonded nonwoven fabric obtained by subjecting a fiber web obtained by a wet-laid paper method to processing by a chemical bonding method to bond the fibers to themselves.

Examples of the fibers included in the fibrous sheet A include natural fibers and synthetic fibers. Especially preferred are thermoplastic fibers, which are a kind of synthetic fibers, from the standpoints that thermoplastic fibers can be produced by melt spinning and that thermoplastic resins have excellent spinnability. It is preferable that the fibrous sheet A includes thermoplastic fibers as a main component. The expression "as a main component" means that thermoplastic fibers are contained in an amount exceeding 50% by mass based on the whole fibrous sheet A, which is taken as 100% by mass. The content of the thermoplastic fibers is more preferably 60% by mass or higher, even more preferably 80% by mass or higher, especially preferably 100% by mass.

Examples of thermoplastic resins for constituting the thermoplastic fibers include polyesters, polyamides, polyolefins, acrylics, Vinylon, polystyrene, and poly(lactic acid). A suitable thermoplastic resin can be selected according to applications, etc. A plurality of thermoplastic resins may be used in combination.

With respect to the fiber diameter of the fibers to be used in the fibrous sheet A, a suitable value thereof may be selected in accordance with the air permeability and collection efficiency which are desired in applications where the air filter medium is to be used as air filters. However, since it is preferable that the fibrous sheet A has high stiffness, the diameter of the fibers used in the fibrous sheet A is preferably 0.1 $\mu$m to 500 $\mu$m. The lower limit thereof is more preferably 1 $\mu$m or larger. The upper limit thereof is more preferably 300 $\mu$m or less, even more preferably 100 $\mu$m or less. By regulating the fiber diameter so as to be within that range, the fibrous sheet A can be prevented from having reduced air permeability and can be made to have enhanced stiffness.

The basis weight of the fibrous sheet A included in the air filter medium of the present invention is preferably 5-500 g/m$^2$. The lower limit thereof is more preferably 10 g/m$^2$ or larger. The upper limit thereof is more preferably 200 g/m$^2$ or less.

By regulating the basis weight of the fibrous sheet A to 5 g/m$^2$ or larger, the fibrous sheet A is made to have higher strength and excellent processability in pleating, honeycombing, etc., and an air filter medium which has low pressure loss can be obtained. Meanwhile, by regulating the basis weight of the fibrous sheet A to 500 g/m$^2$ or less, the air filter medium can be inhibited from having too large a thickness and use of this filter medium in, for example, a pleated filter is effective in inhibiting the pleated filter from suffering an increase in pressure loss due to the thickness of the filter medium. In addition, this air filter medium is excellent also in processability such as suitability for pleating.

It is preferable that the fibrous sheet A included in the air filter medium of the present invention has a tensile strength (rupture strength) of 10-250 N/5-cm width, along both of any direction and the direction perpendicular to said direction. The lower limit thereof is more preferably 20 N/5-cm width or higher, even more preferably 30 N/5-cm width or higher. Meanwhile, the upper limit thereof is more preferably 200 N/5-cm width or less.

By regulating the tensile strength (rupture strength) of the fibrous sheet A to 10 N/5-cm width or higher, the air filter medium can be prevented from rupturing. By regulating the tensile strength (rupture strength) of the fibrous sheet A to 250 N/5-cm width or less, the air filter medium which includes this fibrous sheet A can be easily bent into pleats. The values of tensile strength (rupture strength) were measured in accordance with JIS L1913 (year 2010).

The fibrous sheet B is explained below. The fibrous sheet B may be nonwoven fabric, woven fabric, net, paper, or the like. It is preferable that the fibrous sheet B is nonwoven fabric among these, from the standpoint that nonwoven fabric is excellent in terms of the efficiency of dust collection and suitability for pleating. The nonwoven fabric can be obtained, for example, by a conventional method for nonwoven-fabric production.

Examples of nonwoven fabric suitable as the fibrous sheet B include: nonwoven fabric obtained by subjecting a fiber web obtained by a wet-laid paper method and a dry method to processing by a chemical bonding method, thermal bonding method, needle punching method, water-jet punching method, or the like to bond the fibers to themselves; and nonwoven fabric obtained by a direct spinning method such as a spunbonding method or a melt blowing method. However, the nonwoven fabric obtained by the melt blowing method is especially preferred since this nonwoven fabric is extremely thin, includes fibers having a small diameter, and has an excellent dust collection efficiency as stated above. Those nonwoven fabrics can be produced, for example, by conventional production methods.

Examples of the fibers included in the fibrous sheet B include natural fibers and synthetic fibers. Especially preferred are thermoplastic fibers, which are a kind of synthetic fibers, from the standpoints that thermoplastic fibers can be produced by melt spinning and that thermoplastic resins have excellent spinnability. It is preferable that the fibrous sheet B includes thermoplastic fibers as a main component. The expression "as a main component" means that thermoplastic fibers are contained in an amount exceeding 50% by mass based on the whole fibrous sheet B, which is taken as 100% by mass. The content of the thermoplastic fibers is more preferably 60% by mass or higher, even more preferably 80% by mass or higher, especially preferably 100% by mass.

Examples of thermoplastic resins for constituting the thermoplastic fibers include polyesters, polyamides, polyolefins, acrylics, Vinylon, polystyrene, and poly(lactic acid). A suitable thermoplastic resin can be selected according to applications, etc. A plurality of thermoplastic resins may be used in combination.

With respect to the fiber diameter of the fibers to be used in the fibrous sheet B, a suitable value thereof may be selected in accordance with the air permeability and collection efficiency which are desired in applications where the air filter medium is to be used as air filters. However, the diameter thereof is preferably 0.1 $\mu$m to 500 $\mu$m. The lower limit thereof is more preferably 1 $\mu$m or larger. The upper limit thereof is more preferably 300 $\mu$m or less, even more preferably 100 $\mu$m or less.

By regulating the fiber diameter so as to be within that range, the fibrous sheet B can be prevented from having reduced air permeability and can be made to have enhanced strength. From the standpoint of enabling the fibrous sheet B to be highly excellent in terms of dust collection efficiency, the diameter of the fibers used in the fibrous sheet B is

preferably 20 $\mu$m or less, especially preferably 10 $\mu$m or less.

The basis weight of the fibrous sheet B is preferably 5-500 g/m$^2$. The lower limit thereof is more preferably 10 g/m$^2$ or larger. The upper limit thereof is more preferably 200 g/m$^2$ or less, especially preferably 100 g/m$^2$ or less.

By regulating the basis weight of the fibrous sheet B to 5 g/m$^2$ or larger, the fibrous sheet B is made to have a further improved collection efficiency and the product life of the air filter medium is further prolonged. Meanwhile, by regulating the basis weight of the fibrous sheet B to 500 g/m$^2$ or less, the air filter medium can be inhibited from having too large a thickness and use of this filter medium in, for example, a pleated filter is effective in inhibiting the pleated filter from suffering an increase in pressure loss due to the thickness of the air filter medium. In addition, this air filter medium can be made excellent also in processability such as suitability for pleating.

It is preferable that the fibrous sheet B is nonwoven electret fabric. In cases when the fibrous sheet B is nonwoven electret fabric, an electrostatic dust-collecting effect is added and an air filter medium which has an exceedingly high collection efficiency is obtained.

In the case where the fibrous sheet B is nonwoven electret fabric, it is preferable that the fibers used in the fibrous sheet B include thermoplastic fibers as a main component, from the standpoint of chargeability. The expression "as a main component" means that thermoplastic fibers are contained in an amount exceeding 50% by mass based on the whole fibers constituting the fibrous sheet B, which are taken as 100% by mass. The content of the thermoplastic fibers is more preferably 60% by mass or higher, even more preferably 80% by mass or higher. Meanwhile, there is no particular upper limit thereon, and all the fibers constituting the nonwoven electret fabric may be thermoplastic fibers.

It is preferable that the thermoplastic resin to be used as the thermoplastic fibers included in the fibers which constitute the fibrous sheet B is a polyolefin-based resin, such as polypropylene, from the standpoint that such a resin has a high electrical resistivity and is apt to be charged.

Next, methods for charging the fibrous sheet B are not particularly limited, and a suitable one can be selected at will from among known methods such as a corona discharge method and a pure-water contact method. It is preferred to use the pure-water contact method because fibrous sheets having high electrostatic force and a low pressure loss can be obtained therewith.

As stated above, it is preferable that the fibrous sheet B is melt-blown nonwoven electret fabric including polyolefin-based thermoplastic fibers, from the standpoint of further improving the collection efficiency of the air filter medium.

Meanwhile, the melt-blown nonwoven electret fabric is especially flammable among the nonwoven fabrics mentioned above, since the melt-blown fabric includes flammable polyolefin-based thermoplastic fibers having an extremely small diameter and because no flame retardant has usually been incorporated thereinto in order to avoid a decrease in electret property. Consequently, air filter media which employ melt-blown electret fabric tend to have reduced flame retardancy, although highly excellent in terms of collection efficiency.

However, since the air filter medium of the present invention employs heat-expandable particles capable of imparting excellent flame retardancy to air filter media even when used in a small addition amount, this filter medium has excellent flame retardancy while retaining an excellent collection efficiency, even in the case where the fibrous sheet B is melt-blown nonwoven electret fabric including mainly polyolefin-based thermoplastic fibers.

Namely, the air filter medium of the present invention in which the fibrous sheet B is melt-blown nonwoven electret fabric including polyolefin-based thermoplastic fibers has an excellent collection efficiency, a low pressure loss, and excellent flame retardancy.

Here, an explanation is given on the case where the fibrous sheet B includes two sheets of nonwoven fabric. It is preferable that one of the two sheets of nonwoven fabric is nonwoven fabric (hereinafter referred to as "thin-fiber layer") configured of fibers having a small diameter and that the remainder of the two sheets of nonwoven fabric is nonwoven fabric (hereinafter referred to as "thick-fiber layer") configured of fibers having a diameter larger than the diameter of the fibers constituting the thin-fiber layer.

Thin-fiber layers are prone to be damaged by external force such as friction. An air filter medium is hence configured by superposing a thick-fiber layer, which is less apt to be damaged by external force such as friction, on that surface of a thin-fiber layer which is on the reverse side from the surface in contact with the fibrous sheet A. Thus, that surface of the thin-fiber layer which is on the reverse side from the surface in contact with the fibrous sheet A can be inhibited from suffering fluffing or breakage.

Meanwhile, by interposing a thick-fiber layer between a thin-fiber layer and the fibrous sheet A, the thin-fiber layer can be inhibited from being clogged by dust particles of large sizes. This air filter medium can hence be made to have a further prolonged product life. In order to produce these two effects simultaneously, a fibrous sheet B may be made to have a three-layer structure configured of a thin-fiber layer and thick-fiber layers superposed on both surfaces of the thin-fiber layer.

Next, the binder used in the air filter medium of the present invention is explained. The air filter medium of the present invention has at least one interlaminar space fixed with a binder. The presence of an interlaminar space fixed with a binder renders the strength of adhesion between the fibrous sheets excellent. In addition, since the heat-expandable particles contained in the binder are fixed with the binder to the air filter medium, the heat-expandable particles are inhibited from shedding from the air filter medium. In the case where the air filter medium of the present invention has two or more interlaminar spaces therein, it is preferable that all the interlaminar spaces are fixed with a binder, from the standpoint that the strength of adhesion between the fibrous sheets can be further heightened.

It is preferable that the binder to be used in the air filter medium of the present invention is thermally adhesive particles or thermally adhesive fibers. By using a binder having either of these forms, a plurality of fibrous sheets can be bonded with a small number of bonding sites present in the interlaminar space(s) therebetween, thereby obtaining a stack without impairing air permeability. As the thermally adhesive particles or thermally adhesive fibers, use can be made of ones constituted of a conventionally known synthetic resin.

In the case where the binder is thermally adhesive particles, the average particle diameter of the thermally adhesive particles is preferably 50 $\mu$m to 800 $\mu$m. The lower limit thereof is more preferably 100 $\mu$m or larger, even more preferably 200 $\mu$m or larger. The upper limit thereof is preferably 600 $\mu$m or less, more preferably 400 $\mu$m or less. In cases when the average particle diameter thereof is within that range, not only the particles are inhibited from making a dust of fine powder and show satisfactory handleability but also satisfactory adhesiveness can be maintained.

In the case where the binder is thermally adhesive fibers, examples thereof include: thermoplastic fibers including a single resin component having a lower melting point than the other fibers; and composite fibers which have, in the surface of the fibers, a single resin component having a lower melting point than the other fibers.

With respect to the composite fibers, examples thereof include composite fibers in which the cross-sectional shape is of the core/sheath type in which the fiber surface includes a resin component having a low melting point or of the side-by-side type. With respect to the materials thereof, there are composite fibers constituted of a combination of fiber-forming polymers, such as a copolyester/polyester, copolypropylene/polypropylene, polypropylene/polyamide, polyethylene/polypropylene, polypropylene/polyester, or polyethylene/polyester combination. With respect to the proportion of the thermally adhesive fibers in the whole air filter medium, the thermally adhesive fibers can be used in any desired proportion unless the effect of the invention is lessened thereby.

It is preferable that the low-melting-point resin component included in the binder includes a thermoplastic resin or a thermosetting resin. Examples thereof include: olefin homopolymers such as polyethylene, polypropylene, polybutene, polystyrene, polybutadiene, and polyisoprene and various polyolefin copolymers; copolymers of ethylene with an $\alpha$-olefin having 4 or more carbon atoms (linear low-density polyethylene), ethylene/(meth)acrylic acid compound copolymers such as ethylene/acrylic acid copolymers, ethylene/methyl acrylate copolymers, ethylene/ethyl acrylate copolymers, and ethylene/methacrylic acid copolymers, ethylene/vinyl acetate copolymers (EVA), saponification products or ionomers of ethylene/vinyl acetate copolymers, and mixtures of these resins; and thermoplastic-resin homopolymers and copolymers, such as poly(vinyl chloride)-based resins, acrylonitrile/butadiene/styrene copolymers (ABS resins), acrylonitrile/styrene copolymers, nylons, polyacetal-based resins, acrylic resins, polycarbonate-based resins, polyester-based resins, poly(vinyl acetate)-based resins, poly(vinyl alcohol)-based resins, and polyurethane-based resins and mixtures of these resins.

Preferred are polyolefin-based resins, polyester-based resins, and ethylene/vinyl acetate copolymers (EVA), from the standpoints of imparting film-forming properties, flexibility, and low-temperature processability, bringing about a relatively low cost, and suitability for general-purpose use. Especially preferred are polyolefin-based resins.

A synthetic resin in the form of an aqueous solution or aqueous emulsion can also be used as the binder. However, in cases when a binder in such a form is used, there is a tendency that the pores possessed by the fibrous sheets are undesirably filled with the binder to impair the low pressure loss which is characteristic of the air filter medium of the present invention. Use thereof is hence undesirable.

The low-melting-point resin component included in the binder has a melt flow rate (MFR: g/10 min), which indicates the melt flowability, of preferably 30-300 g/10 min. The lower limit thereof is more preferably 50 g/10 min or higher, even more preferably 80 g/10 min or higher. The upper limit thereof is more preferably 200 g/min or less. By regulating the melt flow rate so as to be within that range, the heat-expandable particles are more tenaciously bonded to the air filter medium in cases when the binder is intended to hold the heat-expandable particles.

The values of melt flow rate (MFR: g/10 min) were measured by a method according to JIS K 7210 (year 2014). Representative examples according to the measuring method are polyethylene (temperature, 190°C; load, 2.16 kgf) and polypropylene (temperature, 230°C; load, 2.16 kgf).

Methods for fixing the interlaminar space between adjacent fibrous sheets with a binder are not particularly limited, and conventionally known methods can be used. Examples thereof include: a method in which particles (thermally adhesive particles) including a thermally adhesive resin are disposed in the interlaminar space between adjacent fibrous sheets and the resultant stack of the fibrous sheets is heated and press-bonded; and a method in which fibers (thermally adhesive fibers) including a thermally adhesive resin are incorporated into fibrous sheets, which are then bonded to each other with an embossing roll.

Next, functional particles usable in the air filter medium of the present invention are explained. In cases when the air filter medium has functional particles therein, it is possible to impart a function, such as the function of removing unpleasant odors or antibacterial or antifungal properties, to the air filter medium. It is hence preferable that the air filter medium includes functional particles disposed in at least one of the interlaminar spaces between adjacent fibrous sheets.

In this case, since the functional particles are disposed in at least one of the interlaminar spaces between adjacent fibrous sheets, the functional particles can be more effectively inhibited from shedding from the air filter medium. In the case where the air filter medium includes three or more stacked fibrous sheets, i.e., where the air filter medium has two or more interlaminar spaces, it is preferable that the functional particles are disposed in the interlaminar space which has the heat-expandable particles and the binder therein. This is because a mixture of the heat-expandable particles and the functional particles can be applied en bloc to an air filter medium during the production and an improvement in production efficiency can hence be attained.

Examples of the functional particles include deodorant particles, photocatalyst particles, antibacterial particles, antifungal particles, antiviral particles, insect-proofing particles, insect pest repellent particles, and fragrance particles. Two or more of these kinds of functional particles shown above as examples may be used in combination. A basis weight of the functional particles can be suitably designed so that the function thereof is exhibited.

In the case where deodorant particles, for example, are used as the functional particles, odors which may be harmful to indoor environments, such as volatile organic compounds, can be adsorptively removed. Use of deodorant particles is hence preferred. As the deodorant particles, use can be made of particles constituted of one of various organic materials, inorganic materials, and organic/inorganic composites or a combination of two or more thereof.

Examples of the organic materials includes synthetic deodorant particles, membranes, resin foam, and porous fibers which are constituted of various polymers. Examples of the inorganic materials include carbon black, coal, activated carbon, various zeolites, various silica gels, alumina, mesoporous silica, silicoaluminophosphate type molecular sieves, and aluminophosphate type molecular sieves, and further include foamed metals, porous metal oxides, metal salts, and clay minerals. Examples of the organic/inorganic composites include composites having a metal/organic compound framework which is called MOF and composites containing organic molecules disposed in intercalation compounds.

Especially useful among these are activated carbon and porous silica gel. Activated carbon is preferred because this material shows an adsorptive effect on an extremely wide range of gases and has an excellent adsorption rate. The activated carbon is not particularly limited so long as harmful gases can be removed therewith. Examples of usable carbonaceous materials for the activated carbon include: plant materials such as fruit shells (e.g., coconut shells and walnut shells), wood, sawdust, charcoal, fruit seeds, by-products of pulp production, lignin, and blackstrap molasses; mineral materials such as peat, peat moss, lignite, brown coal, bituminous coal, anthracite, coke, coal tar, coal pitch, and petroleum distillation residues; synthetic materials such as phenolic resins, Saran resins, and acrylic resins; coals from fibrous materials such as regenerated fibers (e.g., rayon), coconut-shell-derived activated carbon, and wood-derived activated carbon; synthetic materials such as phenolic resins, Saran resins, and acrylic resins; and fibrous materials such as regenerated fibers (e.g., rayon).

It is especially preferred to use coconut shell activated carbon, which has a high hardness and has a large amount of micropores, among those activated carbons.

The hardness herein represents hardness measured by the activated-carbon test method as provided for in JIS K 1474 (year 2014). The hardness is preferably 90% or higher, especially preferably 95% or higher. By using activated carbon having a hardness within that range, the activated carbon is inhibited from being reduced into a fine powder during the processing of the sheets or during pleating, and the shedding of a fine activated-carbon powder from the surfaces of the filter medium or from the ridges of the pleats can be prevented.

The particles of the activated carbon have a BET specific surface area of preferably 500 $m^2/g$ or larger, more preferably 1,000 $m^2/g$ or larger. By regulating the BET specific surface area thereof so as to be within that range, the filter medium can be made to have improved gas-removing ability and be inhibited from increasing in air permeation resistance. The shape of the activated carbon is not particularly limited, and the activated carbon may be particulate (in the form of powder particles), fibrous, etc.

For measuring the BET specific surface area of the particles of activated carbon, conventionally known methods can be used. One example thereof is explained below. First, an about 100-mg portion is taken as a sample out of the activated-carbon particles, vacuum-dried at 180°C for 2 hours, and then weighed. Next, using automatic specific surface area meter Gemini 2375, manufactured by Micromeritics Corp., the sample is examined for nitrogen gas adsorption at the boiling point of liquid nitrogen (-195.8°C) at forty points of relative pressure while gradually elevating the relative pressure in the range of 0.02-1.00. A nitrogen adsorption isotherm of the sample is drawn, and the results corresponding to the relative pressures of 0.02-1.00 are subjected to BET plotting. Thus, the BET specific surface area per unit weight ($m^2/g$) of the activated-carbon particles can be determined.

It is also preferable that the deodorant particles should have undergone an adhesion treatment with a chemical. For example, an amine-based chemical, such as ethanolamine, polyethyleneimine, aniline, or hydrazine, or an alkali ingredient, such as sodium hydroxide, potassium carbonate, or guanidine phosphate, may be fixed or adhered to deodorant particles for the purpose of improving the ability to adsorb acidic or basic gases. Thus, the ability to adsorb acidic gases such as aldehyde-based gases, nitrogen oxides, sulfur oxides, and acetic acid can be improved.

Meanwhile, by fixing or adhering an acidic chemical such as phosphoric acid, citric acid, malic acid, or ascorbic acid to deodorant particles, the ability to adsorb basic gases such as ammonia, methylamine, and trimethylamine can be improved.

Methods for fixing chemicals are not particularly limited, and the chemicals can be fixed by ordinary methods. A suitable method can be selected in accordance with a chemical to be fixed, from among, for example, a method in which deodorant particles are immersed in an aqueous solution containing a chemical to be fixed, a method in which an aqueous solution containing a chemical to be adhered is sprayed, and a method in which deodorant particles are brought into contact with a gas containing a chemical to be adhered. Two or more chemicals may be fixed to deodorant particles. Furthermore, two or more kinds of deodorant particles to which these chemicals have been fixed may be mixed together and used. These techniques can be selected in accordance with the kinds and composition of the gases to be removed.

The number-average particle diameter of the deodorant particles is preferably 100 $\mu$m to 800 $\mu$m, and the lower limit thereof is more preferably 150 $\mu$m or larger, especially preferably 200 $\mu$m or larger. The upper limit thereof is more preferably 500 $\mu$m or less. By regulating the number-average particle diameter thereof so as to be within that range, the deodorant particles can be prevented from shedding from the fibrous sheets and the pressure loss can be prevented from increasing. In addition, the air filter medium can be inhibited from having too large a thickness, and pleated filters and the like obtained therefrom can be inhibited from suffering an increase in filter pressure loss due to the thickness of the filter medium. This filter medium further has excellent suitability for post-processing such as pleating.

The number-average particle diameter of deodorant particles is a value obtained by examining the particles with a scanning electron microscope (SEM) to measure the maximum diameter and minimum diameter of each particle, taking the average of these as the particle diameter, and arithmetically averaging such particle diameters of 100 particles.

From the standpoints of effectively attaining both a low pressure loss and deodorizing performance, it is also preferred to make the deodorant particles have a narrow particle-diameter distribution.

In the case of using deodorant particles as the functional particles in the air filter medium, the basis weight of the deodorant particles is preferably 10-1,000 g/m². The lower limit thereof is preferably 20 g/m² or larger. The upper limit thereof is preferably 800 g/m² or less, more preferably 600 g/m² or less.

By regulating the basis weight thereof to 10 g/m² or larger, deodorizing function can be imparted to the air filter medium in many cases. Meanwhile, by regulating the basis weight of the deodorant particles to 1,000 g/m² or less, the air filter medium can be inhibited from having an increased pressure loss and from having an increased thickness. Pleated filters and the like obtained from the filter medium can hence be inhibited from suffering an increase in pressure loss due to the thickness of the air filter medium. This air filter medium further has excellent suitability for processing such as pleating.

In the air filter medium of the present invention, at least one of the interlaminar spaces between the adjacent fibrous sheets has heat-expandable particles and a binder therein, and the heat-expandable particles in the interlaminar space have been tenaciously fixed with the binder to the air filter medium to inhibit the heat-expandable particles from shedding from the air filter medium. Because of this, in cases when functional particles have been further disposed in this interlaminar space, these functional particles also have been fixed to the air filter medium with the binder like the heat-expandable particles and are hence inhibited from shedding from the air filter medium.

In the case where the air filter medium includes three or more stacked fibrous sheets and has two or more interlaminar spaces therein and where functional particles have been disposed in an interlaminar space which is not the interlaminar space having heat-expandable particles and a binder therein, it is preferable that the interlaminar space in which the functional particles have been disposed has also been fixed with a binder. By employing this embodiment, since the functional particles in the interlaminar space where the functional particles have been disposed are fixed to the air filter medium with a binder, the functional particles are inhibited from shedding from the air filter medium.

From the standpoint of inhibiting the heat-expandable particles and the functional particles from shedding from the air filter medium, it is preferable that the content of the binder in the interlaminar space of the air filter medium is 10% by mass or higher but less than 100% by mass based on the sum of the ingredients disposed in the interlaminar space, such as the heat-expandable particles, functional particles, binder, and flame retardant, the sum being taken as 100% by mass.

By regulating the content thereof to 10% by mass or higher, the heat-expandable particles, functional particles, etc. can be fixed to the air filter medium and the heat-expandable particles, functional particles, etc. can be more effectively inhibited from shedding from the air filter medium. Meanwhile, by regulating the content thereof so as to be less than 100% by mass, the content of the flammable binder can be reduced and the flame retardancy of the air filter medium can be rendered even higher. The lower limit thereof is more preferably 20% by mass or higher. The upper limit thereof is more preferably 50% by mass or less.

Flame retardants, other than heat-expandable particles, which are usable in the air filter medium are explained next. It is preferable that the air filter medium includes a flame retardant besides the heat-expandable particles. An air filter medium having even higher flame retardancy can be thus obtained.

Examples of the flame retardant include organic flame retardants such as phosphorus compound flame retardants, nitrogen compound flame retardants, halogen compound flame retardants, and composites of these, and inorganic flame retardants such as magnesium hydroxide, aluminum hydroxide, antimony compounds, and silicones.

From the standpoints of environmental preservation, etc., it is more preferred to use a phosphorous compound flame retardant among those flame retardants. Examples of the phosphorus compound flame retardant include phosphoric acid, phosphoric acid esters, ammonium phosphate, guanidine phosphate, melamine phosphate, guanylurea phosphate, and polymers of these compounds.

From the standpoint of improving flame retardancy, it is preferable that at least one of the fibrous sheets included in the air filter medium contains a flame retardant. From the standpoint of inhibiting the fibrous sheets from clogging and of thereby reducing the pressure loss, it is preferred to dispose a flame retardant not in the fibrous sheets but in the interlaminar space(s) of the air filter medium.

For imparting a flame retardant to a fibrous sheet, use can be made of a method selected at will from known methods such as, for example, a method in which flame-retardant fibers having a flame retardant are used and a method in which a flame retardant is incorporated into a binder for bonding fibers for constituting the fibrous sheet. Although the flame retardancy may be attained with one of these methods, two or more methods may be used in combination.

It is preferable that the amount of the flame retardant contained in a fibrous sheet is in the range of 1-30% by mass based on the flame-retardant-containing fibrous sheet, which is taken as 100% by mass. The lower limit thereof is more preferably 5% by mass or larger, especially preferably 10% by mass or larger. The upper limit thereof is more preferably 20% by mass or less.

By regulating the content thereof so as to be within that range, the flame retardancy can be improved and the stiffness of the fibrous sheet can be maintained. In addition, the air permeation resistance can be inhibited from increasing.

In the case of an embodiment in which the flame retardant is disposed in an interlaminar space where the heat-expandable particles and functional particles have been disposed, it is preferable that the content of the flame retardant is in the range of 3-10% by mass based on the sum of the ingredients disposed in the interlaminar space, such as the hest-expandable particles, binder, and flame retardant, the sum being taken as 100% by mass. By regulating the content thereof so as to be within that range, the flame retardancy can be improved and the air permeation resistance can be inhibited from increasing.

The fibrous sheets included in the air filter medium of the present invention can be functional sheets to which a function, such as an antibacterial, antifungal, antiviral, insect-proofing, insecticidal, or insect-pest repellent function, has been imparted. For imparting these functions to fibrous sheets, use can be made of, for example, a method in which such ingredients are incorporated into a material for the fibrous sheets through kneading, a method in which the ingredients are incorporated when the fibrous sheets are formed by a wet-laid paper method, or a method in which the ingredients are applied to the fibrous sheets by coating, printing, impregnation, etc.

Next, a process for producing the air filter medium of the present invention is explained. An example of the production process includes: a step of disposing a mixture including heat-expandable particles and a binder on one surface of a first fabric including at least one fibrous sheet; a step of superposing a second fabric including at least one fibrous sheet on the surface of the first fabric on which the mixture has been disposed, thereby obtaining a stack; and a step of heating and press-bonding the stack at a temperature that is not lower than a melting point of the binder and is not higher than an expansion initiation temperature of the heat-expandable particles.

Here, techniques ordinarily employed by a person skilled in the art can be used for disposing the mixture including heat-expandable particles and a binder on one surface of the first fabric, for superposing the first fabric and the second fabric to obtain a stack, and for heating and press-bonding the stack.

The first fabric and the second fabric each may include only one fibrous sheet or may include a plurality of stacked fibrous sheets. In the case where the first fabric or the second fabric includes a plurality of stacked fibrous sheets, such a stack has at least one interlaminar space between adjacent fibrous sheets. It is preferable that the interlaminar space has a binder therein, from the standpoint of improving the strength of adhesion between the fibrous sheets. The interlaminar space may have heat-expandable particles and/or functional particles therein besides the binder.

It is preferable that the binder to be used in the process for producing the air filter medium is thermally adhesive particles or thermally adhesive fibers as stated hereinabove.

EXAMPLES

The present invention is explained below in more detail by reference to Examples. The following Examples should not be construed as limiting the present invention, and any design modifications according to the gist described herein are included in the technical range of the present invention.

[Measuring Methods]

(1) Number-average particle diameter [$\mu$m] of heat-expandable particles or functional particles

Heat-expandable particles or functional particles were examined with a microscope (SEM) to measure the maximum diameter and minimum diameter of each of the particles, and the average of these was taken as the particle diameter. An arithmetic average of such particle diameters of 100 particles was taken as the number-average particle diameter.

(2) Thickness [mm] of air filter medium

A measurement was made by the method described in JIS L 1913 (year 2010), item 6.1.

(3) Basis weight [g/m$^2$] of air filter medium or fibrous sheet

A measurement was made by the method described in JIS L 1913 (year 2010), item 6.2.

(4) Average fiber diameter [$\mu$m]

A surface of a sheet of nonwoven fabric was photographed with a microscope to measure the width of each of 100 fibers. These fiber widths were arithmetically averaged, and this average was taken as the average fiber diameter.

(5) Pressure loss [Pa] of air filter medium

A flat-plate-shaped air filter medium was set inside the wind tunnel having an effective inlet area of 0.03 m$^2$. Air was passed therethrough at a wind velocity of 10.8 cm/sec, and the resultant difference in pressure between the upstream side and the downstream side of the sheet was measured with Manostar Gage.

(6) Dust collection efficiency (%) of air filter medium

A flat-plate-shaped air filter medium was set in a holder having an effective inlet area of 0.1 m$^2$. Air was passed vertically therethrough at a surface wind velocity of 10.8 cm/sec and a dust mixture as provided for as Class 15 in JIS Z 8901 (year 2006) was supplied from the upstream side in a concentration of 70 mg/m$^3$, during which the difference in pressure between the upstream side and the downstream side of the filter was measured with a differential pressure gauge. The dust collection efficiency was calculated using the following equation from the amount of the dust (W1) supplied until the pressure loss increased by 150 Pa from the initial pressure loss and the change in mass (W2) of the air filter medium which occurred during the period. This measurement was made on five samples arbitrarily taken out of each specimen, and the average value thereof was used.

$$\text{Dust collection efficiency (\%)} = (W2/W1) \times 100$$

(7) Deodorization efficiency [%] of air filter medium

A flat-plate-shaped air filter medium was attached to a duct for experiment, and air having a temperature of 23°C and a humidity of 50% RH was sent into the duct at a velocity of 0.1 m/sec. Furthermore, toluene was added to the upstream-side air from a standard gas bomb so as to result in an upstream-side concentration of 70 ppm. The air was sampled on the upstream side and the downstream side of the sheet, and the toluene concentrations of these samples were measured over time using an infrared absorption type continuous monitor. The deodorization efficiency concerning toluene and the toluene adsorption capacity were determined from the measured values.

$$\text{Deodorization efficiency (\%)} = [(C0-C)/C0] \times 100$$

In the equation, C0 is the toluene concentration on the upstream side (= 80 ppm), and C is the toluene concentration on the downstream side (ppm). The deodorization efficiency determined at 3 minutes after initiation of the toluene addition was taken as initial deodorization efficiency. In the Examples, the case where the deodorization efficiency was 60% or higher was rated as high in deodorizing performance.

(8) Flame retardancy of air filter medium

The flame retardancy levels according to "Flammability of Interior Materials" as provided for in FMVSS 302 (JIS D1201 (year 1998)) were used as a standard. In the Examples, the case where the flame did not reach the marked line is indicated by "A"; the case where the flame reached the marked line and the combustion speed was 100 mm/min or lower is indicated by "B"; and the case where the flame reached the marked line and the combustion speed exceeded 100 mm/min is indicated by "C".

(Fibrous sheet I)

Wet-laid-paper-method nonwoven fabric including 10% by mass short polyester fibers having a fineness of 3 dtex, 10% by mass short polyester fibers having a fineness of 1.3 dtex, 15% by mass short Vinylon fibers having a fineness of 6.7 dtex, 36% by mass short Vinylon fibers having a fineness of 17.0 dtex, and 30% by mass acrylic resin binder containing melamine phosphate, and having a basis weight of 46 g/m$^2$ was obtained as fibrous sheet I. The content of melamine phosphate in this sheet was 15% by mass based on the whole fibrous sheet I. Namely, fibrous sheet I is a lowly flame-retardant support having a lower flame-retardant content as compared with fibrous sheet III, which will be described later.

(Fibrous sheet II)

Wet-laid-paper-method nonwoven fabric including 12.5% by mass short polyester fibers having a fineness of 3 dtex, 12.5% by mass short polyester fibers having a fineness of 1.3 dtex, 17% by mass short Vinylon fibers having a fineness of 6.7 dtex, 45% by mass short Vinylon fibers having a fineness of 17.0 dtex, and 13% by mass acrylic resin binder, and having a basis weight of 37 g/m$^2$ was obtained as fibrous sheet II. Namely, fibrous sheet II is a non-flame-retardant support containing no flame retardant.

(Fibrous sheet III)

Wet-laid-paper-method nonwoven fabric including 7.5% by mass short polyester fibers having a fineness of 3 dtex, 7.5% by mass short polyester fibers having a fineness of 1.3 dtex, 9% by mass short Vinylon fibers having a fineness of 6.7 dtex, 27% by mass short Vinylon fibers having a fineness of 17.0 dtex, and 49% by mass acrylic resin binder containing melamine phosphate, and having a basis weight of 62 g/m$^2$ was obtained as fibrous sheet III. The content of melamine phosphate in this sheet was 30% by mass based on the whole fibrous sheet III. Namely, fibrous sheet III is a highly flame-retardant support having a higher flame-retardant content as compared with fibrous sheet I.

(Fibrous sheet IV)

A polypropylene resin was formed into fabric by the spunbonding method so as to result in an average fiber diameter of 20 $\mu$m, a basis weight of 15 g/m$^2$, and a thickness of 0.15 mm. Namely, fibrous sheet IV is spunbonded nonwoven fabric (SB).

(Fibrous sheet V)

Use was made of melt-blown nonwoven electret fabric ("Toraymicron (R)", manufactured by Toray Fine Chemical Co. Ltd.) including polypropylene fibers and having an average fiber diameter of 6 $\mu$m, a basis weight of 30 g/m$^2$, and a thickness of 0.20 mm. Namely, fibrous sheet V is melt-blown nonwoven fabric (MB).

[Example 1]

(Fibrous sheet A) Fibrous sheet I
(Fibrous sheet B) Fibrous sheet IV
(Heat-expandable particles)

An expandable graphite having a number-average particle diameter of 300 $\mu$m and an expansion initiation temperature of 300°C was used as heat-expandable particles.

(Air filter medium)

Thermally adhesive particles made of polyethylene (hereinafter referred to as "polyethylene particles") and the heat-expandable particles were diffused on the fibrous sheet A in amounts of 5 g/m$^2$ and 8 g/m$^2$, respectively, and the fibrous sheet B was superposed thereon. This stack was passed through between two rotating rolls heated at 120°C and the gage spacing was set at 0.7 mm, thereby obtaining an air filter medium which had a basis weight of 74 g/m$^2$ and a thickness of 0.65 mm. In this air filter medium, the content of the heat-expandable particles was 11% by mass based on the whole filter medium, and the content of the binder disposed in the interlaminar space was 38% by mass based on the sum of the ingredients disposed in the interlaminar space, such as a flame retardant, the sum being taken as 100% by mass.

[Example 2]

An air filter medium was produced in the same manner as in Example 1, except that fibrous sheet V was used as the fibrous sheet B. The filter medium had a basis weight of 89 g/m$^2$ and a thickness of 0.66 mm. In this air filter medium, the content of the heat-expandable particles was 9% by mass based on the whole filter medium, and the content of the binder disposed in the interlaminar space was 38% by mass based on the sum of the ingredients disposed in the interlaminar space, such as a flame retardant, the sum being taken as 100% by mass.

[Example 3]

(Fibrous sheet A) Fibrous sheet I
(Fibrous sheet B) Fibrous sheet IV

(Functional Particles)

A particulate activated carbon having a number-average particle diameter of 270 μm was used as functional particles.

(Heat-expandable particles)

An expandable graphite having a number-average particle diameter of 300 μm and an expansion initiation temperature of 300°C was used as heat-expandable particles.

(Air filter medium)

The functional particles, the polyethylene particles, and the heat-expandable particles were diffused on the fibrous sheet A in amounts of 200 g/m$^2$, 60 g/m$^2$, and 8 g/m$^2$, respectively, and the fibrous sheet B was superposed thereon. This stack was passed through between two rotating rolls heated at 120°C and the gage spacing was set at 0.9 mm, thereby obtaining an air filter medium which had a basis weight of 329 g/m$^2$ and a thickness of 0.95 mm. In this air filter medium, the content of the heat-expandable particles was 2% by mass based on the whole filter medium, and the content of the binder disposed in the interlaminar space was 22% by mass based on the sum of the ingredients disposed in the interlaminar space, such as a flame retardant, the sum being taken as 100% by mass.

[Example 4]

An air filter medium was produced in the same manner as in Example 3, except that fibrous sheet V was used as the fibrous sheet B. The filter medium had a basis weight of 344 g/m$^2$ and a thickness of 1.01 mm. In this air filter medium, the content of the heat-expandable particles was 2% by mass based on the whole filter medium, and the content of the binder disposed in the interlaminar space was 22% by mass based on the sum of the ingredients disposed in the interlaminar space, such as a flame retardant, the sum being taken as 100% by mass.

[Example 5]

An air filter medium was produced in the same manner as in Example 4, except that fibrous sheet II was used as the fibrous sheet A. The filter medium had a basis weight of 335 g/m$^2$ and a thickness of 1.02 mm. In this air filter medium, the content of the heat-expandable particles was 2% by mass based on the whole filter medium, and the content of the binder disposed in the interlaminar space was 22% by mass based on the sum of the ingredients disposed in the interlaminar space, such as a flame retardant, the sum being taken as 100% by mass.

[Example 6]

An air filter medium was produced in the same manner as in Example 4, except that the amount of the heat-expandable particles was changed to 16 g/m$^2$. The filter medium had a basis weight of 352 g/m$^2$ and a thickness of 1.04 mm. In this air filter medium, the content of the heat-expandable particles was 5% by mass based on the whole filter medium, and the content of the binder disposed in the interlaminar space was 22% by mass based on the sum of the ingredients disposed in the interlaminar space, such as a flame retardant, the sum being taken as 100% by mass.

[Example 7]

An air filter medium was produced in the same manner as in Example 4, except that the amount of the heat-expandable particles was changed to 2 g/m$^2$. The filter medium had a basis weight of 338 g/m$^2$ and a thickness of 0.99 mm. In this air filter medium, the content of the heat-expandable particles was 1% by mass based on the whole filter medium, and the content of the binder disposed in the interlaminar space was 23% by mass based on the sum of the ingredients disposed in the interlaminar space, such as a flame retardant, the sum being taken as 100% by mass.

[Example 8]

An air filter medium was produced in the same manner as in Example 4, except that an expandable graphite having a number-average particle diameter of 150 $\mu$m and an expansion initiation temperature of 180°C was used as the heat-expandable particles. The filter medium had a basis weight of 344 g/m$^2$ and a thickness of 1.01 mm. In this air filter medium, the content of the heat-expandable particles was 2% by mass based on the whole filter medium, and the content of the binder disposed in the interlaminar space was 22% by mass based on the sum of the ingredients disposed in the interlaminar space, such as a flame retardant, the sum being taken as 100% by mass.

[Comparative Example 1]

An air filter medium was produced in the same manner as in Example 1, except that no heat-expandable particles were used. The filter medium had a basis weight of 66 g/m$^2$ and a thickness of 0.65 mm. The content of the binder disposed in the interlaminar space was 100% by mass based on the sum of the ingredients disposed in the interlaminar space, such as a flame retardant, the sum being taken as 100% by mass.

[Comparative Example 2]

An air filter medium was produced in the same manner as in Example 2, except that no heat-expandable particles were used. The filter medium had a basis weight of 81 g/m$^2$ and a thickness of 0.65 mm. The content of the binder disposed in the interlaminar space was 100% by mass based on the sum of the ingredients disposed in the interlaminar space, such as a flame retardant, the sum being taken as 100% by mass.

[Comparative Example 3]

An air filter medium was produced in the same manner as in Example 3, except that no heat-expandable particles were used. The filter medium had a basis weight of 321 g/m$^2$ and a thickness of 1.01 mm. The content of the binder disposed in the interlaminar space was 23% by mass based on the sum of the ingredients disposed in the interlaminar space, such as a flame retardant, the sum being taken as 100% by mass.

[Comparative Example 4]

An air filter medium was produced in the same manner as in Example 4, except that no heat-expandable particles were used. The filter medium had a basis weight of 336 g/m$^2$ and a thickness of 1.02 mm. The content of the binder disposed in the interlaminar space was 23% by mass based on the sum of the ingredients disposed in the interlaminar space, such as a flame retardant, the sum being taken as 100% by mass.

[Comparative Example 5]

An air filter medium was produced in the same manner as in Example 4, except that fibrous sheet III was used as the fibrous sheet A and that no heat-expandable particles were used. The filter medium had a basis weight of 352 g/m$^2$ and a thickness of 1.10 mm. The content of the binder disposed in the interlaminar space was 23% by mass based on the sum of the ingredients disposed in the interlaminar space, such as a flame retardant, the sum being taken as 100% by mass.

The configurations of the air filter media of Examples 1 to 8 and Comparative Examples 1 to 5 and the results of evaluation thereof are shown in Tables 1, 2, and 3.

Table 1

| | | | Example 1 | Example 2 | Example 3 | Example 4 |
|---|---|---|---|---|---|---|
| Constituent member | Fibrous sheet A | | fibrous sheet I | fibrous sheet I | fibrous sheet I | fibrous sheet I |
| | | | lowly flame-retardant support | lowly flame-retardant support | lowly flame-retardant support | lowly flame-retardant support |
| | Fibrous sheet B | | fibrous sheet IV | fibrous sheet V | fibrous sheet IV | fibrous sheet V |
| | | | SB *1 | MB *2 | SB *1 | MB *2 |
| | Deodorant particles | Material | - | - | activated carbon with no adherent chemical | activated carbon with no adherent chemical |
| | | Basis weight (g/m$^2$) | - | - | 200 | 200 |
| | | Average particle diameter ($\mu$m) | - | - | 270 | 270 |
| | Heat-expandable particles | Material | expandable graphite | expandable graphite | expandable graphite | expandable graphite |
| | | Content (mass%) | 11 | 9 | 2 | 2 |
| | | Average particle diameter ($\mu$m) | 300 | 300 | 300 | 300 |
| | Binder in interlaminar space | Material | polyethylene particles | polyethylene particles | polyethylene particles | polyethylene particles |
| | | Content (mass%) | 38 | 38 | 22 | 22 |

(continued)

|  |  | Example 1 | Example 2 | Example 3 | Example 4 |
|---|---|---|---|---|---|
| Air filter medium | Basis weight (g/m$^2$) | 74 | 89 | 329 | 344 |
| | Thickness (mm) | 0.65 | 0.66 | 0.95 | 1.01 |
| | Dust collection efficiency (%) | 80 | 98 | 80 | 98 |
| | Flame retardancy | A | A | A | A |
| | Pressure loss (Pa) | 8 | 13 | 13 | 25 |
| | Deodorization efficiency (%) | - | - | 92 | 93 |
| *1 SB: spunbonded nonwoven fabric | | | | | |
| *2 MB: melt-blown nonwoven fabric | | | | | |

Table 2

|  |  |  | Example 5 | Example 6 | Example 7 | Example 8 |
|---|---|---|---|---|---|---|
| Constituent member | Fibrous sheet A | | fibrous sheet II | fibrous sheet I | fibrous sheet I | fibrous sheet I |
| | | | non-flame-retardant support | lowly flame-retardant support | lowly flame-retardant support | lowly flame-retardant support |
| | Fibrous sheet B | | fibrous sheet V | fibrous sheet V | fibrous sheet V | fibrous sheet V |
| | | | MB *2 | MB *2 | MB *2 | MB *2 |
| | Deodorant particles | Material | activated carbon with no adherent chemical | activated carbon with no adherent chemical | activated carbon with no adherent chemical | activated carbon with no adherent chemical |
| | | Basis weight (g/m$^2$) | 200 | 200 | 200 | 200 |
| | | Average particle diameter ($\mu$m) | 270 | 270 | 270 | 270 |
| | Heat-expandable particles | Material | expandable graphite | expandable graphite | expandable graphite | expandable graphite |
| | | Content (mass%) | 2 | 5 | 1 | 2 |
| | | Average particle diameter ($\mu$m) | 300 | 300 | 300 | 150 |
| | Binder in interlaminar space | Material | polyethylene particles | polyethylene particles | polyethylene particles | polyethylene particles |
| | | Content (mass%) | 22 | 22 | 23 | 22 |

(continued)

| | | Example 5 | Example 6 | Example 7 | Example 8 |
|---|---|---|---|---|---|
| Air filter medium | Basis weight (g/m$^2$) | 335 | 352 | 338 | 344 |
| | Thickness (mm) | 1.02 | 1.04 | 0.99 | 1.01 |
| | Dust collection efficiency (%) | 98 | 98 | 98 | 98 |
| | Flame retardancy | B | A | B | B |
| | Pressure loss (Pa) | 22 | 25 | 25 | 25 |
| | Deodorization efficiency (%) | 91 | 92 | 92 | 91 |
| *1 SB: spunbonded nonwoven fabric | | | | | |
| *2 MB: melt-blown nonwoven fabric | | | | | |

Table 3

| | | | Comparative Example 1 | Comparative Example 2 | Comparative Example 3 | Comparative Example 4 | Comparative Example 5 |
|---|---|---|---|---|---|---|---|
| Constituent member | Fibrous sheet A | | fibrous sheet I | fibrous sheet I | fibrous sheet I | fibrous sheet I | fibrous sheet III |
| | | | lowly flame-retardant support | lowly flame-retardant support | lowly flame-retardant support | lowly flame-retardant support | highly flame-retardant support |
| | Fibrous sheet B | | fibrous sheet IV | fibrous sheet V | fibrous sheet IV | fibrous sheet V | fibrous sheet V |
| | | | SB *1 | MB *2 | SB *1 | MB *2 | MB *2 |
| | Deodorant particles | Material | - | - | activated carbon with no adherent chemical | activated carbon with no adherent chemical | activated carbon with no adherent chemical |
| | | Basis weight (g/m²) | - | - | 200 | 200 | 200 |
| | | Average particle diameter (μm) | - | - | 270 | 270 | 270 |
| | Heat-expandable particles | Material | - | - | - | - | - |
| | | Content (mass%) | - | - | - | - | - |
| | | Average particle diameter (μm) | - | - | - | - | - |
| | Binder in interlaminar space | Material | polyethylene particles | polyethylene particles | polyethylene particles | polyethylene particles | polyethylene particles |
| | | Content (mass%) | 100 | 100 | 23 | 23 | 23 |

EP 3 275 525 B1

|  |  | Comparative Example 1 | Comparative Example 2 | Comparative Example 3 | Comparative Example 4 | Comparative Example 5 |
|---|---|---|---|---|---|---|
| Air filter medium | Basis weight (g/m$^2$) | 66 | 81 | 321 | 336 | 352 |
|  | Thickness (mm) | 0.65 | 0.65 | 1.01 | 1.02 | 1.10 |
|  | Dust collection efficiency (%) | 80 | 97 | 80 | 97 | 98 |
|  | Flame retardancy | C | C | C | C | B |
|  | Pressure loss (Pa) | 8 | 13 | 13 | 25 | 30 |
|  | Deodorization efficiency (%) | - | - | 92 | 91 | 90 |
| *1 SB: spunbonded nonwoven fabric *2 MB: melt-blown nonwoven fabric |  |  |  |  |  |  |

The air filter media of Examples 1 and 2, which included heat-expandable particles disposed in the interlaminar space between the fibrous sheet A and the fibrous sheet B, showed excellent flame retardancy. Meanwhile, the air filter media of Comparative Examples 1 and 2, which employed the same configurations except for the nonuse of heat-expandable particles, showed poorer flame retardancy than the air filter media of Examples 1 and 2.

The air filter media of Examples 3 to 8, in which deodorant particles serving as functional particles, heat-expandable particles, and a binder had been disposed in the interlaminar space between the fibrous sheet A and the fibrous sheet B, had a deodorizing function. In each of these air filter media, the content (absolute amount) of the flammable binder in the interlaminar space (space between the fibrous sheet A and the fibrous sheet B) where the deodorant particles had been disposed had been increased in order to fix the deodorant particles. Nevertheless, the air filter media of Examples 3 to 8 showed excellent flame retardancy because the filter media had the heat-expandable particles in the interlaminar spaces.

Meanwhile, the air filter media of Comparative Examples 3 and 4, which employed the same configurations except for the nonuse of heat-expandable particles, showed poorer flame retardancy than the air filter media of Examples 3 to 8. Comparative Example 5 showed flame retardancy that was better than the flame retardancy of the air filter media of Comparative Examples 3 and 4 since a flame retardant had been incorporated into the fibrous sheet A in a large amount. However, the binder for fixing the large amount of the flame retardant to the fibrous sheet A was contained in a large amount and, as a result, the air filter medium showed an increased pressure loss.

It can be seen from these results that the air filter media of the present invention can have excellent flame retardancy while retaining a low pressure loss. Consequently, the air filter media of the present invention are considered to be suitable for use in filter applications, in particular, in cabin filter applications where flame retardancy is required.

While the invention has been described in detail and with reference to specific embodiments thereof, it will be apparent to one skilled in the art that various changes and modifications can be made therein without departing from the scope of the claims.

INDUSTRIAL APPLICABILITY

The flame-retardant deodorant filter medium of the present invention is advantageously usable as air filter media for air filters for cleaning the interior air of motor vehicles, railroad cars, etc., filters for air cleaners for use in healthy dwelling houses, condominiums where pets are allowed, homes for elderly people, hospitals, offices, etc., filters for air conditioners, air intake/outtake filters for OA appliances, filters for building air conditioning, filters for industrial clean rooms, etc.

DESCRIPTION OF REFERENCE NUMERALS AND SIGNS

1: Air filter medium
2: Unburned portion
3: Burned portion
4: Carbonized wall

**Claims**

1. An air filter medium comprising:

   a plurality of stacked fibrous sheets; and
   heat-expandable particles and a binder disposed in at least one of interlaminar spaces between adjacent layers of the fibrous sheets,
   wherein the heat-expandable particles in an unexpanded state have a number-average particle diameter of 100 μm to 800 μm.

2. The air filter medium according to claim 1, wherein the binder is thermally adhesive particles or thermally adhesive fibers.

3. The air filter medium according to claim 1 or 2, comprising functional particles disposed in at least one of the interlaminar spaces between adjacent layers of the fibrous sheets.

4. The air filter medium according to claim 3, wherein the functional particles are deodorant particles having a number-average particle diameter of 100 μm to 800 μm.

5. The air filter medium according to any one of claims 1 to 4, wherein at least one of the fibrous sheets is nonwoven fabric comprising thermoplastic fibers as a main component.

6. The air filter medium according to any one of claims 1 to 5, wherein at least one of the fibrous sheets is a nonwoven electret fabric.

7. A process for producing an air filter medium, the process comprising:

a step of disposing a mixture comprising heat-expandable particles and a binder on one surface of a first fabric comprising at least one fibrous sheet, wherein the heat-expandable particles in an unexpanded state have a number-average particle diameter of 100 μm to 800 μm;
a step of superposing a second fabric comprising at least one fibrous sheet on the surface of the first fabric on which the mixture has been disposed, thereby obtaining a stack; and
a step of heating and press-bonding the stack at a temperature that is not lower than a melting point of the binder and is not higher than an expansion initiation temperature of the heat-expandable particles.

8. The process for producing an air filter medium according to claim 7, wherein the binder is thermally adhesive particles or thermally adhesive fibers.

**Patentansprüche**

1. Luftfiltermedium, das
eine Vielzahl von gestapelten Faserlagen; und
wärmeexpandierbare Teilchen und ein Bindemittel aufweist, in mindestens einem der interlaminaren Räume zwischen benachbarten Schichten der Faserlagen angeordnet, wobei die wärmeexpandierbaren Teilchen in einem nicht expandierten Zustand einen zahlenmittleren Teilchendurchmesser von 100 μm bis 800 μm haben.

2. Luftfiltermedium nach Anspruch 1, wobei das Bindemittel thermisch klebende Teilchen oder thermisch klebende Fasern ist.

3. Luftfiltermedium nach Anspruch 1 oder 2, das funktionelle Teilchen aufweist, die in mindestens einem der interlaminaren Räume zwischen benachbarten Schichten der Faserlagen angeordnet sind.

4. Luftfiltermedium nach Anspruch 3, wobei die funktionellen Teilchen Deodorantteilchen mit einem zahlenmittleren Teilchendurchmesser von 100 μm bis 800 μm sind.

5. Luftfiltermedium nach einem der Ansprüche 1 bis 4, wobei mindestens eine der Faserlagen ein Vliesstoff ist, der thermoplastische Fasern als Hauptkomponente aufweist.

6. Luftfiltermedium nach einem der Ansprüche 1 bis 5, wobei mindestens eine der Faserlagen ein Elektretvlies ist.

7. Verfahren zur Herstellung eines Luftfiltermediums, wobei das Verfahren einen Schritt des Aufbringens einer Mischung, die wärmeexpandierbare Teilchen und ein Bindemittel aufweist, auf eine Oberfläche eines ersten Gewebes, das mindestens eine Faserlage aufweist, wobei die wärmeexpandierbaren Teilchen in einem nicht expandierten Zustand einen zahlenmittleren Teilchendurchmesser von 100 μm bis 800 μm haben;
einen Schritt, bei dem die Oberfläche des ersten Gewebes, auf dem die Mischung angeordnet wurde, mit einem zweiten Gewebe, das mindestens eine Faserlage aufweist, überlagert wird, wodurch ein Stapel erhalten wird; und
einen Schritt des Erwärmens und Pressbindens des Stapels bei einer Temperatur umfasst, die nicht niedriger als ein Schmelzpunkt des Bindemittels und nicht höher als eine Expansionsinitiierungstemperatur der wärmeexpandierbaren Teilchen ist.

8. Verfahren zur Herstellung eines Luftfiltermediums nach Anspruch 7, wobei das Bindemittel thermisch klebende Teilchen oder thermisch klebende Fasern ist.

**Revendications**

1. Milieu de filtre à air comprenant :

   une pluralité de feuilles fibreuses empilées ; et
   des particules thermo-expansibles et un liant disposés dans au moins l'un des espaces interlaminaires entre des couches adjacentes des feuilles fibreuses,
   dans lequel les particules thermo-expansibles dans un état non expansé ont un diamètre de particule moyen en nombre allant de 100 μm à 800 μm.

2. Milieu de filtre à air selon la revendication 1, dans lequel le liant est constitué de particules thermo-adhésives ou de fibres thermo-adhésives.

3. Milieu de filtre à air selon la revendication 1 ou 2, comprenant des particules fonctionnelles disposées dans au moins l'un des espaces interlaminaires entre des couches adjacentes des feuilles fibreuses.

4. Milieu de filtre à air selon la revendication 3, dans lequel les particules fonctionnelles sont des particules déodorantes ayant un diamètre de particule moyen en nombre allant de 100 μm à 800 μm.

5. Milieu de filtre à air selon l'une quelconque des revendications 1 à 4, dans lequel au moins l'une des feuilles fibreuses est un tissu non tissé comprenant des fibres thermoplastiques en tant que composant principal.

6. Milieu de filtre à air selon l'une quelconque des revendications 1 à 5, dans lequel au moins l'une des feuilles fibreuses est un tissu électret non tissé.

7. Procédé de production d'un milieu de filtre à air, le procédé comprenant :

   une étape de disposition d'un mélange comprenant des particules thermo-expansibles et un liant sur une surface d'un premier tissu comprenant au moins une feuille fibreuse, où les particules thermo-expansibles dans un état non expansé ont un diamètre de particule moyen en nombre allant de 100 μm à 800 μm ;
   une étape de superposition d'un deuxième tissu comprenant au moins une feuille fibreuse sur la surface du premier tissu sur lequel le mélange a été disposé, obtenant ainsi un empilement ; et
   une étape de chauffage et de liaison par pression de l'empilement à une température qui n'est pas inférieure à un point de fusion du liant et n'est pas supérieure à une température d'initiation d'expansion des particules thermo-expansibles.

8. Procédé de production d'un milieu de filtre à air selon la revendication 7, dans lequel le liant est constitué de particules thermo-adhésives ou de fibres thermo-adhésives.

## Fig. 1

## Fig. 2

**EP 3 275 525 B1**

**REFERENCES CITED IN THE DESCRIPTION**

**Patent documents cited in the description**

- JP 2004358389 A
- JP 10212685 A
- JP 2000126523 A
- WO 2009055047 A1